# EUROPEAN PATENT APPLICATION

(11) **EP 3 398 584 A1**
(43) Date of publication of application: **07.11.2018**
(21) Application number: 16880209.8
(22) Date of filing: 28.12.2016
(51) Int. Cl.: A61K 8/26, A61K 8/27, A61Q 15/00

(54) **COSMETIC COMPOSITIONS COMPRISING A SYNERGISTIC COMBINATION OF PERSPIRATION-ABSORVING AND PERSPIRATION-ADSORBING PARTICLES, AND USE THEREOF**

(30) Priority: 30.12.2015 US 201514984661
(71) Applicant: Natura Cosméticos S.A., 05106-000 São Paulo SP (BR)
(72) Inventor: CANALLI ORTIZ, Giovanna, 05106-000 São Paulo - SP (BR); SPINA, Marcos Rogério, 05106-000 São Paulo - SP (BR); RODRIGUES DE PAULA, Leonardo, 05106-000 São Paulo - SP (BR); ANGELINO DOS SANTOS TEODORO, Silvania, 05106-000 São Paulo - SP (BR); RIBEIRO MOURA, Cybele, 05106-000 São Paulo - SP (BR); GANDINI ANDRÉO, Luciana, 05106-000 - SP (BR)
(74) Representative: Abel & Imray
(86) International application number: PCT/BR2016/050359
(87) International publication number: WO 2017/112997

(57) **Abstract**

The present invention provides cosmetic compositions useful to protect against humidity and to act against perspiration, comprising a synergistic combination of perspiration-absorbing and perspiration-adsorbing particles.

## Description

### Field of the invention

The present invention relates to cosmetic compositions useful in protection to moisture and in resistance to sweat, comprising a synergistic combination of sweat absorbent and adsorbent particles.

### Technical problem

Body deodorant and antiperspirant compositions are widely used for the purpose of reducing excess sweat and bodily odors.

Sweat is a bodily phenomenon of great physiological importance, enabling dissipation of heat from the organism through change in water's phase from liquid to vapor, followed by subsequent evaporation. This is an extraordinarily efficient method of preventing hyperthermia, that is to say, overheating of the body, thus enabling the human beings to live at extreme temperatures and under adverse conditions of temperature.

Sweat secretion is a substrate for the bacterial metabolic activity, which originates volatile products responsible for the identity of bodily odor, which is considered unpleasant in many situations.

Although the sweat produced by the human skin does not have significant odor, the fermentation thereof by the bacteria present on the skin produces compounds with odor that is considered unpleasant, especially trans-3-methyl-2-hexenoic acid. The amount of water in the armpit region is vital to the metabolic activity of microorganisms. Thus, the less the moisture in this region the less proliferation of the microorganisms there will be and, as a result, the production of bad smell. For this reason, body deodorant compositions are generally formulated with components that have sweat reducing activity, such as the so-called antiperspirants.

The mechanism of action of antiperspirants is the diffusion of salt through the sweat-duct, which after the slow neutralization of the acidic solution of metal salt, produces a gel or mucopolysaccharide complex. This temporary obstruction prevents sweat from coming out and remains until the affected keratin has been replaced by the normal processes of cell renewal.

The present invention provides a cosmetic composition that offers an alternative and complementary mode of protection against sweat, that is to say, it acts without modifying the natural perspiration process, imparting protection against sweat, dry protection and greater resistance to sweat.

### Summary of the invention

The present invention provides cosmetic compositions comprising combinations of adsorbent and absorbent particles that surprisingly exhibit synergistic effect in protection against sweat (perspiration), as well as the use thereof and methods for protection against sweat.

### Detailed description of the present invention

Cosmetic compositions for protection against sweat, which comprise a combination of absorbent and adsorbent particles that surprisingly exhibit a synergistic effect in protection against sweat, as well as the use thereof and methods for protection against sweat.

According to the present invention, a synergistic combination of particles comprising at least zinc oxide and hydrophobic mica treated with amino acid exhibit a synergistic effect in protecting against sweat, without interfering in the natural process of perspiration.

Preferably, the treatment of mica particles treated with amino acid is made with lauroyl lysine.

Optionally, the combination of particles of the present invention may further comprise one or more components selected from the group comprising silica, acrylic derivatives, talc, starches, and mica with hydrophobic and/or lipophobic treatment, preferably with fluorine derivatives.

Preferably, the compositions of the present invention comprise about 0.5% to about 8% zinc oxide, which imparts adsorption to sweat and helps in the antimicrobial protection, and about 0.5% to about 15% mica with hydrophobic treatment, preferably mica treated with amino acid for greater resistance to sweat and greater adherence of the product to the skin. Additionally, the compositions of the present invention optionally comprise about 5% to about 80% of talc or starch or mica with hydrophobic and lipophobic treatment derived from fluorine, about 0.0% to about 5% porous silica and/or about 0.0% to about 3% acrylic polymers that potentiate the synergistic effect of absorbing and adsorbing sweat.

Beside the combination of sweat adsorbent and absorbent particles, the compositions of the present invention optionally may further comprise additional actives such as deodorant actives: ethylhexyl glycerin, 2-methyl 5-cyclohexylpentanol, polyglyceryl 3 caprylate, other deodorant actives: c12-c13 alkyl lactate, sodium bicarbonate, farnesol, ricinoleate, zinc gluconates, triethyl citrate, zinc PCA, propanediol, polyglycerils, caprilyl glycol, etc.

The compositions of the present invention may be formulated in various forms known in the art, as for example, powder, emulsion, gel, creme, rollon, stick, aerosol, among other cosmetic preparations.

As a person skilled in the art knows, the compositions of the present invention may further contain carriers, preservatives, emollients, excipient wetting agents such as: glycerin, oils and esters, butters, gums, bentone, stearic acid derivatives, etc.

Besides body deodorant compositions, the combination of particles of the present invention may also provide a method of protection to sweat which can be applied in other areas of the body, as skin and hair susceptible to sweat.

As used herein, the term "talc" comprises steatite, a phyllosilicate mineral with chemical composition Mg₃Si₄O₁₀(OH)₂, generally in the form of fibrous or sheet masses. Phyllosilicates or sheet-silicates form parallel sheets of silicate tetrahedrons with Si₂O₅ or a ratio of 2:5. Their structure contributes to the sweat-adsorbing properties with a dry touch and pleasant feeling. As used herein, the term "mica" is a highly lamellar mineral, including various closely related minerals, of the group of phylosilicates, which have a highly perfect basal division. All of them are monoclinic crystals, with a tendency to pseudo-hexagonal, and are similar in their chemical composition. The highly perfect division, which is the most prominent characteristic of mica, is explained by the hexagonal arrangement of its atoms along successively parallel planes. Its structure contributes to the sweat adsorbing properties and greater adhesiveness of the particles to the skin.

As used herein, term "starch", as for example: maize starch, rice starch, tapioca starch, comprises a carbohydrate mainly constituted by glucose with glycoside bonds produced by green plants as an energy reservoir. Preferably, the starch used is tapioca starch which has truncated spherical particles. Its structure contributes to the sweat absorbing properties and of greater adhesiveness of the particles to the skin with silky feeling without leaving a whitish aspect on the skin.

As used herein, the term "silica" comprises silicon dioxide, forming porous amorphous particles with ellipsoid shape and exhibiting an extensive surface area that causes it to absorb both oil and water. Preferably it has capacity to absorb 5 to 6 times its weight. Its structure contributes to sweat absorbing properties with a dry touch and pleasant feeling.

As used herein, the term "zinc oxide" comprises an inorganic compound with formula ZnO. Its structure contributes to the sweat adsorbing properties and antimicrobial action.

As used herein, the term "acrylic polymers" comprises superabsorbent polymers, which preferably can absorb more than 100 times their weight in water. Their structure contributes to the sweat adsorbing properties.

### Examples

### Example 1

**Starch powder cosmetic forms; mica treated with lauroyl lysine; zinc oxide and porous silica;**
**talc, mica treated with lauroyl lysine; zinc oxide and porous silica;**
**Mica treated with fluorine derivative, mica treated with lauroyl lysine; zinc oxide and acrylic derivative**
Durability of powdered formulations

For each evaluation time (0, 2, 8, 12, and 24 hours) one prepared and pre-hydrated 6 workpieces of Vitro-Skin® (IMS Testing Group, EUA) with useful area of 35 mm x 23 mm, to which 1mg cm-2 mass of the sample of the product under study was applied. Digitized initial images were obtained on white and black backgrounds. Then, the workpieces with the applied sample were subjected to the durability test simulated in climatic chamber, for 2h at 35 ± 2°C and 90% ± 5% RH. After the simulation time, the workpieces were digitized again on the white and black backgrounds. The procedure was repeated for the times of 8, 12 and 24 hours. From the initial images obtained, and at each simulation time, the initial and after immersion opacities were calculated. The durability was determined as a function of the maintenance of opacity (equivalent to the opening) of the VitroSkin with the applied product after the simulation time of durability with respect to the initial one.

The powdered formulations exhibited long duration characteristics, exhibiting durability of up to 24 hours from application.

**POWDERED FORMULAS**

| Code lims | Description | <2111.23274.4> (%) | <2111.23274.6> (%) | <2111.23274.7> (%) |
|---|---|---|---|---|
| 16620 | 2-methyl 5-cyclohexylpentanol | 0.4 | 0.4 | 0.4 |
| 19610 | Sodium polyacrylate | 0 | 0 | 1 |
| 1149 | Dimeticone and trimethylsiloxysilane | 3.5 | 3.5 | 3.5 |
| 6439 | ethylhexylglycerin | 0.5 | 0.5 | 0.5 |
| | Mica and perfluorooctyl trietoxysilane | 0 | 0 | 78.6 |
| 964 | Mica and lauroyl lysine | 10 | 10 | 10 |
| 157 | silica | 2 | 2 | 0 |
| 10229 | Sodium benzoate/gluconolactone | 1 | 1 | 1 |
| 1177 | Tapioca starch | 77.6 | 0 | 0 |
| 4703 | Talc | 0 | 77.6 | 0 |
| 11074 | Zinc oxide | 5 | 5 | 5 |
| | | 100 | 100 | 100 |

### Example 2

### Protection against sweat (perspiration of volunteers)

Initially the volunteers selected underwent 7 days' conditioning, without using any deodorant, antiperspirant or any other product in the armpits.

After the conditioning period, the volunteers returned for 4 consecutive days for standardized application of the investigation product.

Prior to application, the volunteers were told to wash their armpits with standardized soap.

The product was applied to one armpit, and the opposite armpit was used as control, without application of product.

### Powdered formulation comprising starch; mica treated with lauroyl lysine; zinc oxide and porous silica

On average, 79.5% of the volunteers noticed that the armpit treated with the investigation product exhibited greater feeling of protection against sweat.

On average, 79.5% of the volunteers said that the armpit treated with the investigation product perspired less.

On average 75.6% of the volunteers found that the armpit treated with the investigation product had a more pleasant feeling.

On average, 76.9% of the volunteers felt that the armpit treated with the investigation product had drier skin.

On average,79.5% of the volunteers stated that the investigation product provided "dry protection".

On average, 84.6% of the volunteers stated that the investigation product "protected against sweat".

### Powdered formulation comprising talc; mica treated with lauroyl lysine; zinc oxide, and porous silica

On average, 90.6% of the volunteers noticed that the treated armpit exhibited greater feeling of protection against sweat.

On average, 97.9% of the volunteers said that the treated armpit perspired less.

On average, 95.8% of the volunteers found that the treated armpit had a more pleasant feeling.

On average, 86.5% of the volunteers felt that the treated armpit had a drier skin.

On average, 90.6% of the volunteers stated that the product provided "dry protection".

On average, 94.8% of the volunteers stated that the product "protected against sweat".

### Powdered formulation comprising mica with hydrophobic and lipophobic treatment; Mica treated with lauroyl lysine; Zinc oxide and acrylic polymers

On average, 75% of the volunteers noticed that the armpit treated with the investigation product presented greater protection against sweat.

On average, 69% of the volunteers said that the armpit treated with the investigation product perspired less.

On average, 65.5% of the volunteers found that the armpit treated with the investigation product had a more pleasant feeling.

On average, 60.7% of the volunteers felt that the armpit treated with the investigation product provided a drier skin.

On average, 64.3% of the volunteers stated that the investigation product provided "dry protection".

On average, 69.0% of the volunteers stated that the investigation product "protected against sweat".

### Gel crème formulation with concentration of 20% of the combination of particles in gel crème formulation comprising talc; mica treated with lauroyl lysine; zinc oxide and porous silica.

On average, 80.6% of the volunteers noticed that the armpit treated with the investigation product provided greater feeling of protection against sweat.

On average, 80.6% of the volunteers said that the armpit treated with the investigation product perspired less.

On average, 75.0% of the volunteers found that the armpit treated with the investigation product provided a more pleasant feeling.

On average, 59.3% of the volunteers felt that the armpit treated with the investigation product provided a drier skin.

On average, 73.1% of the volunteers stated that the investigation product provided a "dry protection".

On average, 83.3% of the volunteers stated that the investigation product "protected against sweat".

**GEL CREME FORMULA**

| Description | <2111.24491.5> (%) |
|---|---|
| 2-methyl-5-cyclohexylpentanol | 0.4 |
| Aqua (or water) | 71.05 |
| Dimeticone and trimethylsiloxysilicate | 1.5 |
| Dihydroacetic acid | 0.6 |
| disodium EDTA | 0.05 |
| Ethylhexylglycerin | 0.5 |
| Glycerin | 3 |
| Lauroyl lysine and mica | 2 |
| Phenoylethanol | 0,9 |
| Silica | 0.4 |
| Copolymer Hydroxyethyl acrylate/acryloyldimethyl sodium taurate; squalane, water, polysorbate 60, sorbitan isostearate | 1.5 |
| Talc | 16.6 |
| Xanthan gum | 0.5 |
| Zinc oxide | 1 |
| | 100 |

### Emulsion formulation with concentration of 20% combination of particles in water-in-oil emulsion comprising talc; mica treated with lauroyl lysine; zinc oxide and porous silica - texture: emulsion

91.7% of the volunteers noticed that the armpit treated with the investigation product exhibited greater feeling of protection against sweat.

78.3% of the volunteers said that the armpit treated with the investigation product perspired less.

80.0% of the volunteers found that the armpit treated with the investigation product provided more pleasant feeling.

76.7% of the volunteers felt that the armpit treated with the investigation product provided drier skin.

83.3% of the volunteers stated that the investigation product provided "dry protection".

76.7% of the volunteers stated that the investigation product "protected against perspiration".

**EMULSION FORMULA**

| Description | <2111.24601.3> (%) |
|---|---|
| 2-methyl 5-cyclohexylpentanol | 0.4 |
| Aqua (or water) | 39.75 |
| Cyclopentanesiloxane/dimeticone crosspoli | 5 |
| Cetyl peg/ppg-10/1 dimeticone 90 | 2.5 |
| cyclopentasiloxane | 20.5 |
| Cyclopentanesiloxane/propylene carbonate | 2 |
| Dmdm hydantoin | 0.6 |
| Ethylhexylglycerin | 0.5 |
| Glycerin | 5 |
| Lauroyl lysine and mica | 2 |
| Polyglyceryl-4 isostearate | 2.5 |
| silica | 0.4 |
| silica | 0.5 |
| Sodium chloride | 0.75 |
| talc | 16.6 |
| Zinc oxide | 1 |
| | 100 |

### Evaluation of the perception of the volunteer for subjective attributes after each application of the product after dry sauna; comparison with product with aluminum salt Powdered formulation comprising talc; mica treated with lauroyl lysine; zinc oxide and porous silica

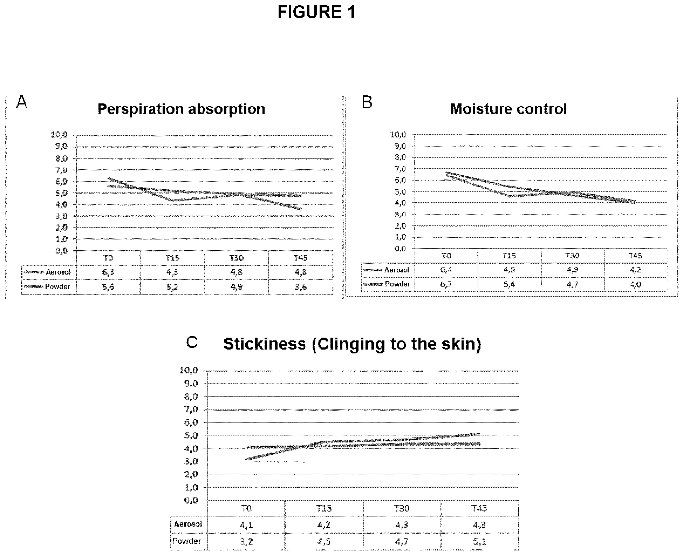

Figure 1A shows the mark given with respect to the absorption of sweat (absorption of the perspiration) by the volunteers to the armpit with the composition of the present invention and with a traditional deodorant composition.
Figure 1B shows the mark given with respect to the moisture control (dry skin) by the volunteer to the armpit with the composition of the present invention and with a traditional deodorant composition.
Figure 1C shows the mark given with respect to the stickiness to the skin (clinging to the skin) by the volunteer to the armpit with the composition of the present invention and with a traditional deodorant composition.

### Gel crème formulation comprising talc; mica treated with lauroyl lysine; zinc oxide and porous silica

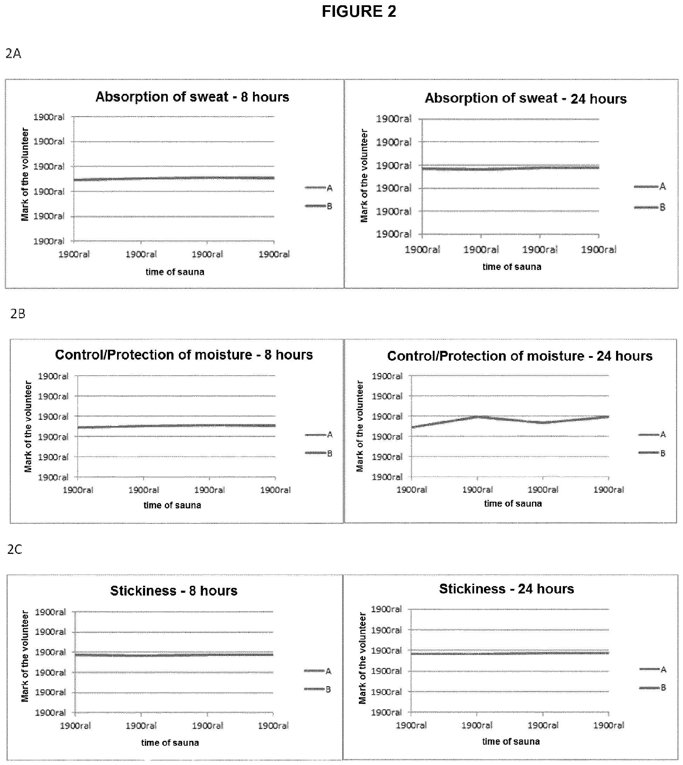

Figure 2A shows the mark given with respect to the absorption of sweat (absorption of perspiration) by the volunteer to the armpit with the composition of the present invention and with a traditional deodorant composition. The stroke A represents the composition according to the present invention, and stroke B traditional antiperspirant.

Figure 2B shows the mark given with respect to the control of moisture (dry skin) by the volunteer to the armpit with the composition of the present invention and with a traditional deodorant composition. Stroke A represents the composition according to the present invention, and stroke B traditional antiperspirant.

Figure 2C shows the mark given with respect to stickiness to the skin (clinging to the skin) by the volunteers to the armpit with the composition of the present invention and with a traditional deodorant composition. Stroke A represents the composition according to the present invention and stroke B traditional antiperspirant.

### Gel crème formulation comprising talc; mica treated with lauroyl lysine; zinc oxide and porous silica - 10% of the mixture of particles

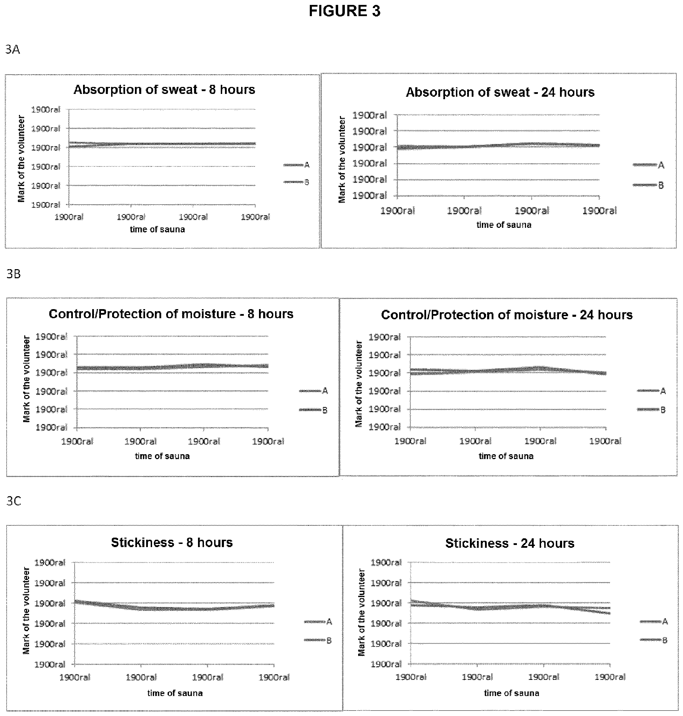

Figure 3A shows the mark given with respect to absorption of sweat (absorption of perspiration) by the volunteer to the armpit with the composition of the present invention and with a traditional deodorant composition. Stroke A represents the composition according to the present invention and stroke B traditional antiperspirant.

Figure 3B shows the mark given with respect to the moisture control (dry skin) by the volunteer to the armpit with the composition of the present invention and with a traditional deodorant composition. Stroke A represents the composition according to the present invention, and stroke B traditional antiperspirant.

Figure 3C shows the mark given with respect to stickiness to the skin (clinging to the skin) by the volunteer to the armpit with the composition of the present invention and a traditional deodorant composition. Stroke A represents the composition according to the present invention, and stroke B traditional antiperspirant.

## Claims

1. A cosmetic composition **characterized by** comprising a combination of particles comprising zinc oxide and mica treated with an amino acid.

2. The composition according to claim 1, **characterized in that** the mica particles are treated with lauroyl lysine.

3. The composition according to claim 1 or 2, **characterized by** further comprising particles of one or more groups consisting of silica, acrylic derivatives, talc, starches, and mica with hydrophobic and/or lipophobic treatment, preferably with fluorine derivatives.

4. The

5. The composition according to any one of claims 1 to 4, **characterized by** comprising about 0.5% to about 8% zinc oxide.

6. The composition according to any one of claims 1 to 4, **characterized by** comprising about 0.5% to about 15% mica treated with amino acid.

7. The composition according to any one of claims 1 to 6, **characterized by** further comprising about 5% to about 80% talc or starch or mica with hydrophobic and lipophobic treatment.

8. The composition according to any one of claims 1 to 6, **characterized by** further comprising about 0.0% to about 5% porous silica.

9. The composition according to any one of claims 1 to 6, **characterized by** further comprising about 0.0% to about 3% acrylic polymers.

10. The composition according to any one of claims 1 to 9, further comprising deodorant components.

11. The composition according to claim 10, **characterized in that** the deodorant components are selected from the group consisting of ethylhexylglycerin, 2-methyl 5-cyclohexylpentanol and polyglyceryl 3 caprylate.

12. Use of a composition as defined in any one of claims 1 to 11, **characterized in that** it is for protection against sweat.

13. A method for protection against sweat, **characterized in that** it comprises applying a composition as defined in any one of claims 1 to 11 in the area to be protected against sweat.
